# EUROPEAN PATENT APPLICATION

(11) **EP 1 287 806 A1**
(43) Date of publication of application: **05.03.2003**
(21) Application number: 01930143.1
(22) Date of filing: 15.05.2001
(51) Int. Cl.: A61K 7/00

(54) **SHEET-TYPE PACKS**

(30) Priority: 15.05.2000 JP 2000141331
(71) Applicant: HISAMITSU PHARMACEUTICAL CO. INC., Tosu-shi, Saga-ken 841-0017 (JP)
(72) Inventor: MUTA, Kazunori, Hisamitsu Pharmaceutical Co., Inc., Tosu-shi Saga 841-0017 (JP); KOSE, Yasuhisa, Hisamitsu Pharmaceutical Co., Inc., Tosu-shi Saga 841-0017 (JP); HIRANO, Munehiko, Hisamitsu Pharmaceutical Co. Inc, Tosu-shi Saga 841-00 17 (JP)
(74) Representative: Vossius, Volker, Dr.
(86) International application number: JP0104026
(87) International publication number: WO01087244

(57) **Abstract**

Sheet-type packs prepared by adding a refreshing agent, a perfume and/or a sebum-controlling agent to a base comprising a water-soluble polymer, polyhydric alcohol, a crosslinking agent, water and a skin-care component. These packs exert favorable effects on the skin, high safety and an excellent effect of relaxing mind and the body while maintaining appropriate stickiness to the skin.

## Description

### Technical Field

The invention relates to a sheet-type pack, which is prepared by adding a refreshing agent, a perfume and/or a sebum-controlling agent to a base comprising a water-soluble polymer, a polyhydric alcohol, a crosslinking agent, water and a skin-care component.

### Background Art

Recently, a pack excellent in water holding capacity having a low removal strength, which contains polyacrylates, polyhydric alcohols and water as the main components, has been described in JP, A, 54-49334 as a pack preparation. Also, in JP, B, 1-46485 a sheet-type pack using a cross-linking type aqueous gel as a base material and further in JP, A, 5-295004 a pack mixed with a naturally derived semisynthetic component as a moisturizing agent and a thickener are disclosed.

However, the conventional packs have a problem that the packing action for the skin is not sufficient and problems that a sense of strangeness such as pressure or smart at the time of sticking tends to arise and that it can not satisfy relaxation needs of users even at the present time when the sheet-cosmetic boom is very active.

Further, in JP, A, 8-283147 an aqueous adhesive composition is disclosed in which lasting and appropriate refreshing sense is given when it is applied to the skin as an external preparation. However, said composition is used only as a component of an external preparation such as a compress sheet and use as a component of a sheet-type pack is not examined at all.

Therefore, it is strongly desired to develop a sheet-type pack which exerts favorable effects on the skin, excellent safety and an effect of relaxing mind and body while maintaining appropriate stickiness to the skin.

### Disclosure of the Invention

The purpose of the invention is to provide a sheet-type pack which solves the above conventional problems and exerts favorable effects on the skin, excellent safety and an effect of relaxing mind and body while maintaining an appropriate stickiness to the skin. More specifically, the purpose is to provide a sheet-type pack which gives fine refreshing feeling when using and a lasting action of the fine refreshing feeling after using, and exerts an excellent effect in a sebum-removal action and a sebum-suppressive action and an excellent effect of relaxing by fine fragrance and comfortable feeling.

After extensive researches to solve the above problems the inventors have found out that these are solved by adding a refreshing agent, a perfume and/or a sebum-controlling agent to each component used in the conventional pack, and thus accomplished the invention.

Namely, the invention relates to a sheet-type pack prepared by adding one or more species selected from the group consisting of refreshing agents, perfumes and sebum-controlling agents to a base comprising a water-soluble polymer, a polyhydric alcohol, a crosslinking agent, water and a skin-care component.

Also, the invention relates to the above sheet-type pack in which the water-soluble polymer comprises one or more species selected from the group consisting of gelatin, polyacrylic acid, partial neutralization products of polyacrylic acid and polyacrylic acid salts.

Further, the invention relates to the above sheet-type pack in which the polyhydric alcohol comprises one or more species selected from the group consisting of polyethylene glycol and polypropylene glycol.

Also, the invention relates to the above sheet-type pack in which the cross-linking agent comprises one or more species selected from the group consisting of a slightly water-soluble aluminum compound and a polyfunctional epoxy compound.

Further, the invention relates to the above sheet-type pack comprising as the refreshing agent one or more species selected from the group consisting of peppermint oil, d-camphor, dl-camphor, l-menthol, isopulegol, 3-L-menthoxypropane-1,2-diol, menthyl pyrrolidonecarboxylate and L-menthyl-3-hydroxybutylate.

Also, the invention relates to the above sheet-type pack comprising as the perfume one or more species selected from a group consisting of eucalyptus oil, dl-menthol and clove oil.

Further, the invention relates to the above sheet-type pack comprising clove oil as the sebum-controlling agent.

Also, the invention relates to the above sheet-type pack wherein a mixing amount of the refreshing agent, the perfume and/or the sebum-controlling agent is 0.001-3 wt.% of the total amount of the base.

Further, the invention relates to the above sheet-type pack wherein an antiseptic is added.

Also, the invention relates to the above sheet-type pack, which is applied to the face.

Further, the invention relates to the above sheet-type pack wherein the sebum component content after use is reduced not more than 40% of that before use.

### Mode for carrying out the Invention

Illustrative of the water-soluble polymers are gelatin, polyacrylic acid or salts thereof, partial neutralization products thereof or the like, and each can be used individually or by mixing more than two species. As the polyacrylic acid salts, salts of metals such as sodium, lithium and potassium are preferable, and one whose average degree of polymerization is 1000-100000 is preferably used. Among these water-soluble polymer bases a mix by a combination of gelatin and partial neutralization products of polyacrylic acid is especially preferable. As the mixing amount of these water-soluble polymers, it is 3-25 wt.%, preferably 5-20 wt.%, and more preferably 5-10 wt.%. As the mixing amount becomes not more than 5 wt.%, adhesiveness, agglutinativeness, shape retaining property, water absorption power and the like, of a preparation is reduced, and it easily produces the tendency to induce non-uniformity of adhesive mass, lowering of workability and lowering of usability, whereby it is unfavorable in less than 3 wt.% because the tendency is remarkable. Also, as the mixing amount becomes not less than 10 wt.%, adhesiveness, agglutinativeness and shape retaining property of the preparation is reduced, and it easily produces the tendency to induce an excessive increase of viscosity during production, non-uniformity of adhesive mass, lowering of workability and lowering of usability, whereby it is unfavorable in more than 25 wt.% because the tendency is remarkable.

The polyhydric alcohol consists of a mix of glycols, the mixing amount based on the total amount of the base being 1-35 wt.%, preferably 5-25 wt.%, and more preferably 5-20 wt.%. As the mixing amount becomes not more than 5 wt.%, the tendency to induce adhesiveness and an agglutinativeness of the preparation, lowering of water absorption power and shape retaining property, non-uniformity of gel before use, lowering of workability and lowering of usability when using, is shown, whereby it is unfavorable in less than 1 wt.% because in particular the tendency is remarkable. Also, as the mixing amount becomes not less than 25 wt.%, adhesiveness and agglutinativeness of the preparation, and shape retaining property and water absorption power are reduced. In addition, the tendency to induce lowering of workability and lowering of usability is observed, whereby it is unfavorable in more than 35 wt.% because in particular the tendency is remarkable. As for the polyhydric alcohol it is most preferable when adjusting the mix to the range of 5-20 wt.%. Further, as the glycols in the polyhydric alcohol, polyethlene glycol of the average molecular weight 200-600, polypropylene glycol of the average molecular weight 500-3000, and the like having an polyether structure are preferable, and one or more species of these can be used by mixing.

As the cross-linking agent, one or more species of a slightly water-soluble aluminum compound or a polyfunctional epoxy compound can be used by mixing. Illustrative of the slightly water-soluble aluminum compounds are aluminum hydroxide, aluminum silicate hydrate, synthetic aluminum silicate, kaolin, aluminum acetate, aluminum lactate, aluminum stearate, dry aluminum hydroxide gel, magnesium metasilicate aluminate, magnesium silicate aluminate, aluminum dihydroxy aminoacetate, synthetic hydrotalcite, aluminum magnesium silicate and the like, and one or more species of these can be used by mixing. Use of the slightly water-soluble aluminum compounds gives a gel appropriate strength in the initial physical property as a filler in adding an inhibitory effect against skin irritancy by the antacid action in addition to the skin astringent action by trace aluminum ion, and along with this, since the aluminum ion dissolves into the preparation in the time course, the function is provided to cover the lowering of the gel strength owing to the time dependent decomposition of the polymer and the time dependent cleavage of the cross-linking part of covalent bondings between polymer molecules. Further, the aluminum dissolution rate can be controlled by adjusting pH.

Illustrative of the polyfunctional epoxy compounds are polyethleneglycol diglycidyl ether, ethyleneglycol diglycidyl ether, glycerin diglycidyl ether, glycerin triglycidyl ether, propyleneglycol diglycidyl ether, polyglycerol polyglycidyl ether, sorbitol polyglycidyl ether, sorbitan polyglycidyl ether, trimethylolpropane polyglycidyl ether, pentaerythrityl polyglycidyl ether, resorcinol diglycidyl ether, neopentylglycol diglycidyl ether and the like.

One or more species of these polyfunctional epoxy compounds can be used by mixing. Excellent water absorption and shape retaining property can be obtained by use of the polyfunctional epoxy compounds, and they can produce covalent bonding with the water soluble polymers having a carboxyl, amino or hydroxyl groups, or the like, enhancing a gel strength. As the mixing amount of these crosslinking agents, that is, the slightly water-soluble aluminum compounds and/or the polyfunctional epoxy compounds, it is used in 0.05-20 wt.%, preferably 0.5-15 wt.%, and more preferably 1-10 wt.%. As the mixing amount becomes not more than 1 wt.%, the tendency to induce lowering of agglutinativeness, the shape retaining property and water absorption power of the preparation, lowering of time dependent stability in the physical property of the preparation, lowering of workability, lowering of safety for the skin and lowering of usability is shown, whereby it is unfavorable in less than 0.05 wt.% because in particular the tendency is remarkable. Also, as the mixing amount becomes not less than 10 wt.%, the tendency to induce adhesiveness, agglutinativeness, shape retaining property, excessive increase of viscosity during production, non-uniformity of adhesive mass by gelation, lowering of workability, lowering of safety for the skin and lowering of usability is shown, whereby it is unfavorable in more than 20 wt.% because in particular the tendency is remarkable. Further, considering physical properties, real feeling of use or the effect of use in the preparation, use of the slightly water-soluble aluminum compound together with the polyfunctional epoxy compound is preferable.

As the water, purified water, sterile water or natural water is used. The water acts as a dispersion-dissolution agent for the skin-care component, the moisturizing component, the water-soluble polymer, the cross-linking agent, the antiseptic and the like, and is especially important to disperse and dissolute the skin-care component and the moisturizing agent uniformly in the preparation. Further, the water itself increases usability during use and after use, and moves into the skin together with the moisturizing agent, bringing an effect to give moisture and tension. Owing to this, the mixing amount of water should be large, it being 60-95 wt.%, preferably 65-90 wt.%, and more preferably 70-85 wt.%. Relative humidity of the preparation itself can be heightened by letting it contain a large amount of water in the preparation, and it becomes possible to drain off effectively a large amount of water to the outside, consequently giving moisture to the skin and depriving the heat of vaporization by the evaporation of the water to the outside, thereby providing comfortable refreshing feeling. As the mixing amount of water becomes not more than 70 wt.%, there is a tendency to induce an adhesiveness of the preparation, lowering of water absorption power before use, lowering of workability and lowering of usability when using, whereby it is unfavorable in less than 60 wt.% because the tendency is remarkable. Also, as the mixing amount becomes not less than 85 wt.%, the adhesiveness and the agglutinativeness are easily inhibited, and there is a tendency to lower the shape retaining property before use, whereby it is unfavorable in more than 95 wt.% because the tendency is remarkable.

Illustrative of the skin-care components are allantoin, horse chestnut extract, water-soluble placenta extract, sage extract, kojic acid, lecithin, amino acids, proteins, saccharides, hormones, placenta extract, or extraction components from various crude drugs such as aloe, loofah and glycyrrhia, or vitamin A, vitamin C, vitamin D, vitamin E and other vitamins or derivatives thereof, or dipotassium glycyrrhizinate, diphenhydramine hydrochloride, diphenhydramine salycilate, diphenhydramine tannate, triprolidine hydrochloride, mequitazine, chlorpheniramine maleate, d-chlorpheniramine maleate, clemastine fumarate, promethazine hydrochloride, tranilast, sodium crompglycate, ketotifen, arylsulfatase B, bufexairiac, bendazac, butyl flufenamate, ibuprofen piconol, indomethacin, aspirin, flurbiprofen, ketoprofen, piroxicam, 2-pyridinemethyl mefenamic acid, 5,6-dehydroarachidonic acid, 5, 6-methano-LTA4, esculetin, eupatilin; caffeic acid or benoxaprofen, and one or more species of these can be used by mixing. As the mixing amount of the skin-care components, it is used in 0.001-10 wt.%, preferably 0.005-5 wt.%, and more preferably 0.01-1 wt.%. As the mixing amount becomes not more than 0.005 wt,%, it easily produces a tendency to induce lowering of workability and lowering of usability and use effect, whereby it is unfavorable in less than 0.001 wt.% because the tendency is remarkable. Further, as the mixing amount becomes not less than 5 wt.%, adhesiveness, agglutinativeness and the shape retaining property of the preparation are reduced, and also it easily produces a tendency to induce non-uniformity of adhesive mass, lowering of workability, lowering of safety, and lowering of usability and use effects, whereby it is unfavorable in more than 10 wt. because the tendency is remarkable.

As the refreshing agent it can be selected from peppermint oil, d-camphor, dl-camphor, isopulegol, 3-L-menthoxypropane-1,2-diol, menthyl pyrrolidonecarboxylate and L-menthyl-3-hydroxybutylate, and one or more species can be mixed. As to the mixing amount, it is 0.0005-2.9 wt.%, preferably 0.0005-2 wt.%, and more preferably 0.001-1 wt.% based on the total amount of the base.

In less than 0.0005 wt.%, it is difficult to obtain the relaxing effect by refreshing fragrance and fine usability. Also, in more than 2.9 wt.%, there is a tendency that not only refreshing effect, also skin-stimulation feeling becomes too strong because a cooling effect becomes especially strong. Further, it is not favorable in view of the cost performance because the problem of cost increase occurs.

As the perfume it can be selected from eucalyptus oil, dl-menthol, clove oil and the like, and one or more species can be mixed. As to the mixing amount, it is 0.0001-2.9 wt.%, preferably 0.0005-2 wt.%, and more preferably 0.001-1 wt.% based on the total amount of the base. In less than 0.0001 wt.%, not only the relaxing effect by refreshing fragrance but also the synergistic relaxing effect by a combination of the refreshing agent are reduced. Also, in more than 2.9 wt.%, the fragrance becomes extremely strong, and there is a tendency inducing unpleasant feeling when using. Further, it is not favorable because problems such as lowering of adhesiveness due to the bleed phenomenon of the perfume component in view of the preparation and increase of cost in view of the cost performance are induced.

As the sebum-controlling agent, clove oil is used. As to the mixing amount, it is 0.00001-2.9 wt.%, preferably 0.00001-2 wt.%, and more preferably 0.00002-1 wt.% based on the total amount of the base. In less than 0.00001 wt.%, a sebum component can not be removed sufficiently. Also, in more than 2.9 wt.%, it is not favorable in usability due to the increase of stickiness or the like.

Further, the mixing amount occupied by one or more species of the refreshing agent selected from peppermint oil, d-camphor, dl-camphor and 1-menthol, one or more species of the perfume selected from eucalyptus oil, dl-menthol and clove oil, and the sebum-controlling agent of clove oil in the total amount of the base is preferably in the range of 0.001-3 wt.%, and it becomes most effective when the ratio of components in these species is adjusted to the range of 0.1-160 : 0.1-110. Namely, it is most appropriate as the combination of the refreshing agent and the perfume and their mix balance because the effect of the skin-care action is remarkably increased by the relaxing effect due to refreshing fragrance and fine usability.

Further, as the sebum-removal action and sebum-suppressive action effects, the mix balance of the components is preferable wherein the ratio of the sebum component per unit area after using the sheet-type pack of the invention is reduced to not more than 40% of that before use, and furthermore preferable when the above ratio becomes not more than 30%.

Further, in the sheet-type pack of the invention can appropriately be mixed in, in addition to the above base components a suitable amount with an antiseptic, moisturizing component, antioxidant, adhesiveness tackifying agent, dissolution agent, pigment, perfume, surfactant, UV absorber, an inorganic filler, pH adjusting agent and the like known per se.

Illustrative of the antiseptics are p-hydroxybenzoic acid ester (for example, methylparaben, ethylparaben, propylparaben), 1,2-pentanediol, benzoic acid, benzoate, salicylate, sorbic acid, sorbate, dehydroacetate, 4-isopropyl-3-methylphenol, 2-isopropyl-5-methylphenol, phenol, hinokitiol, cresol, 2,4,4'-2'-hydroxydiphenylether, 3,4,4'-trichlorocarbanide, chlorobutnol, benzalkonium chloride and benzethonium chloride, and one or more species of these can be used by mixing. Among these p-hydroxybenzoic acid ester is preferable. As the mixing amount, it is used in 0.005-10 wt.%, preferably 0.01-5 wt.%, and more preferably 0.01-1 wt.%. As the mixing amount becomes not more than 0.01 wt,%, there is a tendency to induce putrefaction of the preparation by outbreak of molds or bacteria during storage and lowering of usability when using and after using, whereby it is unfavorable in less than 0.005 wt.% because the tendency is remarkable. Also, as the mixing amount becomes not less than 1 wt.%, there is a tendency to bring a subtle change of adhesiveness and agglutinativeness in the preparation and influences such as usability and unpleasant feeling by irritation and antiseptic smell, whereby it is unfavorable in more than 10 wt.% because the tendency is remarkable.

As the moisturizing component can be mixed one or more species of rose fruit extract, orange extract, orange juice, raspberry extract, kiwi extract, cucumber extract, gardenia extract, grape fruit extract, crataegus fruit extract, common juniper extract, duke extract, tomato extract, grape extract, loofah extract, lime juice, apple extract, apple juice, lemon extract, lemon juice, aqueous succinyl kefiran solution, aqueous acetyl kefiran solution, aqueous maleyl kefiran solution, malt root extract, rose extract, collagen, ceramide, hyaluronic acid, etc. In particular, as to fruit extracts (fruit juices) they have effects as a perfume, and have an action to heighten the effect of the refreshing agent and the perfume that are essential components.

As the antioxidant can be mixed sodium edetate, ascorbic acid, propyl gallate, butylhydroxyanisol, dibutyl hydroxy toluene, nordihydroguaretic acid, tocopherol, tocopherol acetate and the like.

As the tackifying agent can be mixed casein, pullulan, agar, dextran, sodium aluginate, soluble starch, carboxystarch, dextrin, carboxymethyl cellulose, sodium carboxymethyl cellulose, methylcellulose, ethylcellulose, hydroxyethylcellulose, polyvinyl alcohol, polyethylene oxide, polyacrylamide, polyacrylic acid, polyvinylpyrrolidone, carboxyvinyl polymer, polyvinyl ether, polymaleic acid copolymer, methoxyethylene maleicanhydride copolymer, isobutylene maleicanhydride copolymer, polyethylene imine and the like.

As the dissolution agent can be mixed benzyl alcohol, pyrothiodecane, peppermint oil, isopropyl mirystate,crotamitone and the like.

As the pigment can be mixed the officially designated pigments such as Red No.2 (amarnath), Red No.3 (erythrosine), Red No.102 (new coccine), Red No.104-1 (phloxine B), Red No.105-1 (rose bengale), Red No.106 (acid red), Yellow No.4 (tartrazine), Yellow No.5 (sunset yellow FCF), Green No.3 (fast green FCF), Blue No.1 (brilliant blue FCF), Blue No.2 (indigo carmine) and the like. As for the pigments they are not particularly limited, though they give a large influence to the preparation image and result to the improvement of usability and activation feeling to the skin.

As the surfactant can be mixed an anionic surfactant such as dioctyl sodium sulfosuccinate, alkylsulfate, 2-ethylhexyl alkylsulfate sodium salt or sodium n-dodecyl benzenesulfonate, a cationic surfactant such as hexadecyl trimethylammonium chloride, octadecyl dimethyl benzyl ammonium chloride or polyoxyethylene dodecyl monomethylammonium chloride, and a nonionic surfactant such as polyoxyethylene stearylether, polyoxyethylene tridecylether, polyoxyethylene nonylphenylether, polyoxyethylene octylphenylether, polyoxyethylene monostearate, sorbitan monostearate, sorbitan monopalmitate, sorbitan sesquioleate, polyoxyethylene sorbitan monolaurate, polyoxyethylene sorbitan monooleate, glycerol monostearate, polyglycerin fatty acid ester or polyoxyethylene octadecyl amine.

As the UV absorber can be mixed p-aminobenzoic acid, p-aminobenzoate, amyl p-dimethylaminobenzoate, salicilate, methyl anthranilate, umbelliferone, esculin, benzyl silicate, cinoxate, guaiazulene, urocainic acid, 2-(2-hydroxy-5-methylphenyl)benzotriazole, 4-methoxybenzophenone, 2-hydroxy-4-methoxy benzophenone, dioxybenzone, octabenzone, dihydroxy dimethoxybenzophenone, slisobenzone, benzoresorcinol, octyl p-dimetylaminobenzoate, ethylhexyl p-methoxycinnamate, and the like.

As the inorganic filler can be mixed titanium oxide, talc, zinc oxide, silicate hydrate, magnesium carbonate, dibasic calcium phosphate, magnesium silicate, diatomaceous earth, anhydrous silicic acid, bentonite and the like.

As the pH adjusting agent can be mixed acetic acid, formic acid, lactic acid, tartaric acid, oxalic acid, benzoic acid, glycolic acid, malic acid, citric acid, hydrochloric acid, nitric acid, sulfuric acid, sodium hydroxide, potassium hydroxide, methylamine, ethylamine, propylamine, dimethylamine, diethylamine, dipropylamine, trimethylamine, triethylamine, tripropylamine, monomethanolamine, monoethanolamine, monopropanolamine, dimethanolamine, diethanolamine, diprpopanolamine, trimethanolamine, triethanolamine, tripropanolamine and the like.

The pH of the adhesive mass that has been appropriately mixed with a suitable amount of each of the above components is to be controlled not to give irritancy to the skin, and the pH of the adhesive mass 5g diluted with purified water to 100 g is in the range of 5-8, preferably 5.5-7.5, more preferably 6-7.

Further, illustrative of the backing on which the adhesive mass is applied is a flexuous material such as a synthetic resin film of polyethlene, polypropylene, polyethlene terephthalate, ethylene-vinylacetate coplymer, vinyl chloride, polyurethane, polyester, polyamide, rayon, polyester or the like, elastic nonwoven-fabric, nonwoven paper, laminate of nonwoven fabric or nonwoven paper with the above synthetic resin film or sheet, nonwoven fabric such as absorbent cotton or the like, cloth, elastic cloth, paper, cellophane or the like, and it can appropriately be selected according to the application. Further, the pack layer is applied on the backing layer consisting of the flexuous backing and the surface of this pack layer is covered further with a removable film or paper, whereby the stability of the preparation can be kept. Also, as to the removable paper, in order to stick the preparation easily to the face a separation line, perforating or the like are set, making the form easy for separation. Furthermore, color of the backing layer is not particularly limited, though it greatly affects the image of the preparation and provides usability and activation feeling to the skin in a greater degree, white, skin color, yellow, red, orange, green, blue, pink, light blue, brown or the like being cited, and if necessary a shade is preferably adjusted.

In the process for preparing the sheet-type pack of the invention, the above components are uniformly mixed and/or dissolved in a stirring machine and spread on the backing layer of non-dyeing or dyeing, thereon a removable paper is stuck, and it is cut in a shape of the face. Further, parts for the eyes, nose, mouth and tin are cut in an appropriate shape, whereby it is processed for the convenience of handling. Also, aiming to use in a specific part of the face it can be processed into a shape to be applied well to the aimed part as a nose pack which is to be applied to the nose or an eye-around pack which is to be applied to the part around the eye. Further, a sheet-type pack is desirably preserved in a sealed bag or container until use from the view point of prevention from contamination under preservation, decrease of effectiveness by the evaporation of a volatile substance, or the like.

Since the sheet-type packs of the invention are sheet-type packs which are prepared by adding a refreshing agent, a perfume and/or a sebum-controlling agent to the base comprising a water-soluble polymer, a polyhydric alcohol, a crosslinking agent, water and a skin-care component, they exert favorable effects on the skin, high safety and an excellent effect of relaxing mind and the body while maintaining appropriate stickiness to the skin.

### Example

In the following, the sheet-type packs of the invention are explained in more detail by the examples and the test examples. However, the invention is not limited in any way by these.

### (Example 1)

Pigment 0.0015 wt.% is dispersed in purified water 74.195 wt.%. This is dissolved with gelatin 0.5 wt.%, aqueous placenta extract 1 wt.%, ethylparaben 0.1 wt. %, subsequently added with peppermint oil 0.08195 wt.%, L-menthol 0.0035 wt.%, d-camphor 0.81970 wt.%, eucalyptus oil 0.08195 wt.%, dl-menthol 0.00820 wt.%, clove oil 0.00820 wt.%, sorbitol polyglycidyl ether 0.2 wt,.%, further added with a mixture (Table 1) of partial neutralization products of polyacrylic acid 7 wt.%, polyethlene glycol 10 wt.%, polypropylene glycol 5 wt.% and synthetic aluminum silicate 1 wt.%, and stirred till these become homogeneous: Subsequently, the mixture is spread on the backing layer to make the depth about 1.4 mm and is stuck with a film. Further, after sticking, it is cut in a shape of the face, and parts of the eyes, nose, mouth and tin are cut in an appropriate shape to provide the sheet-type pack.

### (Example 2-9)

The sheet-type packs were obtained by the preparation in the same way as example 1 using the mixing agents and the mixing amounts shown in Table 1. Further, the component (%) means wt.%.

### Comparative Example

### (Comparative example 1) * Without mixing of a refreshing agent and perfume

The sheet-type pack was prepared in the same formulation as that of example 1 except that the refreshing agent and the perfume were removed from the formulation of example 1 and the mixing amount of purified water was made to 75.1985 wt.%.

### (Comparative example 2) * Without mixing of a refreshing agent

The sheet-type pack was prepared in the same formulation as that of example 1 except that the refreshing agent was removed from the formulation of example 1 and the mixing amount of purified water was made to 75.10015 wt.%.

### (Comparative example 3) * Without mixing of a perfume

The sheet-type pack was prepared in the same formulation as that of example 1 except that the perfume was removed from the formulation of example 1, and the mixing amount of purified water was made to 74.29335 wt.%.

The components of the above comparative examples 1-3 together with the components of examples 1-9 are shown in Table 1.

**Table 1**

| Components(%) | Examples Examples | | | | | | | | | Comparative examples | | |
|---|---|---|---|---|---|---|---|---|---|---|---|---|
| | 1 | 2 | 3 | 4 | 5 | 6 | 7 | 8 | 9 | 1 | 2 | 3 |
| Peppermint oil | 0.08195 | 0.00033 | 0.0422 | 0.3104 | 0.00016 | 0.00804 | 0.00156 | 0.033 | 0.04545 | | | 0.08195 |
| d-Camphor | 0.81970 | | 0.4219 | 0.1724 | 0.00161 | | | | | | | 0.81970 |
| dl-Camphor | | 0.00040 | | 0.2000 | | 0.48232 | 0.01558 | 0.040 | 0.05456 | | | |
| Eucalyptus oil | 0.08195 | 0.00020 | 1.6878 | 0.0062 | 0.00016 | 0.00804 | 0.00156 | 0.020 | 0.03636 | | 0.08195 | |
| l-Menthol | 0.0035 | | 0.0042 | 0.2104 | 0.00805 | | 0.00015 | 0.004 | | | | 0.0035 |
| dl-Menthol | 0.00820 | 0.00004 | | 0.1000 | | 0.00080 | | | 0.04545 | | 0.0082 | |
| Clove oil | 0.00820 | 0.00003 | 0.8439 | 0.0006 | 0.00002 | 0.00080 | 0.03115 | 0.003 | 0.01818 . | | 0.0082 | |
| Aqueous placenta extract | 1 | 1 | | | 0.09 | 0.5 | | | 3 | 1 | 1 | 1 |
| Allantoin | | 0.05 | 0.1 | 0.04 | | 0.5 | 0.05 | 0.1 | | | | |
| Polyethylene glycol | 10 | 15 | 5 | 15 | | 5 | 1 | 10 | | 10 | 10 | 10 |
| Polypropylene glycol | 5 | 5 | 5 | 20 | 10 | | | | 10 | 5 | 5 | 5 |
| Gelatin | 0.5 | 2 | 1 | 3 | 0.7 | | 3 | 1 | 5 | 0.5 | 0.5 | 0.5 |
| Polyacrylic acid | | | 2 | | | | | | 10 | | | |
| Sodium polyacrylate | | | 4 | 0.9 | 4 | | | | | | | |
| Partial neutralization products | 7 | 6 | | | | 8 | | 6 | 10 | 7 | 7 | 7 |
| Kaolin | | | 4 | | | 8 | | | | | | |
| Aluminum acetate | | | | | 0.05 | 2 | | | | | | |
| Synthetic aluminum silicate | 1 | | 2 | | | | | 4 | | 1 | 1 | 1 |
| Sorbitol polyglycidyl ether | 0.2 | | | 0.05 | | | 0.1 | | | 0.2 | 0.2 | 0.2 |
| Polyethylene glycol diglycidyl ether | | | 0.02 5 | | | | 0.1 | | | | | |
| Propylene glycol diglycidyl ether | | 0.02 | | | | | 0.1 | | | | | |
| Glycerin diglycidyl ether | | | 0.02 5 | | | | | | 0.5 | | | |
| Polyglycerol polyglycidyl ether | | | | | 0.05 | | | 0.05 | | | | |
| Glycerin triglycidyl ether | | 0.08 | | | | | 0.1 | | | | | |
| Methylparaben | | 0.3 | 0.1 | | | | 0.5 | 0.25 | 0.01 | | | |
| Ethylparaben | 0.1 | | 0.05 | | | 0.5 | | | | 0.1 | 0.1 | 0.1 |
| Propylparaben | | | 0.05 | 0.01 | 0.1 | | | | 0.01 | | | |
| Perfume | | 0.001 | | | | | | | 0.0001 | | | |
| Pigmsnt | 0.0015 | | 0.001 | | | | | 0.003 | 0.0005 | 0.0015 | 0.0015 | 0.0015 |
| Water | 74.195 | 70.548 | 73.649 | 60 | 85 | 75 | 95 | 78.497 | 61.2794 | 75.1985 | 75.10015 | 74.29335 |

### Test Example

### (Test example 1) Adhesiveness test

As for examples 4-9 the results of the adhesiveness test carried out are shown in Table 2. In the test was used the samples which were let stand in advance for not less than 30 min. under the condition of 25°C-60%Rh, and the test was carried out under the same condition. First, the sticking part of the sample was fixed on a horizontal stand in the upward direction. Subsequently, a steel ball whose diameter is 20/32 inch was dropped from the height of 17.35 cm for the length of 30 cm on the base in the sine curve and was rolled on the sticky surface. Here, the distance (cm) from the ground point of the steel ball to the reaching point was evaluated as the sticking strength. As for, the steel ball used the material was SUJ2 of JIS 64805 (high carbon chromium bearing steel) and the precision was the upper class of JIS B1501 (steel ball for ball bearing).

**Table 2**

| | Adhesive strength |
|---|---|
| Example 4 | 9 cm |
| Example 5 | 10 cm |
| Example 6 | 3 cm |
| Example 7 | 12 cm |
| Example 8 | 4 cm |
| Example 9 | 15 cm |

As shown in Table 2 each example showed good adhesiveness (Test example 2) Residual test of sebum-component content on the skin surface

As for examples 1,2 and 3 and the comparative examples 1,2 and 3, the sebum-component content on the skin surface were measured before and after use, and the comparing results was each shown in Table 3. In the test, after the subjects were restricted for not less than 30 min. under the condition of 25°C-60%Rh, the amount of the sebum components on the forehead was measured using SKICOS 301 ( manufactured by Amique Group Co., Ltd.). Subsequently, after the samples cut into 3 cm×3 cm were stuck on the forehead of the subjects, the sebum-component amount on of the forehead was measured likewise.

**Table 3**

| | Sebum- component amount | (µg/cm²) |
|---|---|---|
| | Before use | After use |
| Example 1 | 80 | 23 |
| Example 2 | 76 | 20 |
| Example 3 | 82 | 21 |
| Comparative example 1 | 78 | 44 |
| Comparative example 2 | 81 | 37 |
| Comparative example 3 | 77 | 40 |

As shown in Table 3, although in examples 1,2 and 3 the sebum-component amounts per unit area after use were 29 %, 26 % and 26 % respectively, being reduced to not more than 30 % of those before use, in comparative examples 1,2 and 3 they were 56 %, 46 % and 52 % respectively. Therefore, in examples 1,2 and 3 a more remarkable sebum-removal action and sebum-suppressive effect compared with comparative examples 1,2 and 3 was observed.

### (Test example 3) Usability evaluation test

As for examples 8, 2 and 3 and comparative examples 1, 2 and 3 the usability test was carried out. In the test, 30 male persons in the age of twenties were divided into the group for using example 8 and comparative example 1, the group for using example 2 and comparative example 2, and further the group for using example 3 and comparative example 3 (each consisting of 10 persons) and tested. The subjects were provided with one sheet each of both samples of the examples and the comparative examples and made to use each sheet on a different day, and the evaluation was made in 5 degrees from very good to not good regarding the refreshing feeling, the efficacy feeling and the relaxing feeling. The test results are shown in Table 4 (refreshing feeling), in Table 5(efficacy feeling) and in Table 6 (relaxing feeling).

**Table 4**

| [Refreshing feeling] | | | | | |
|---|---|---|---|---|---|
| | Very good | Good | Mediocre | Not very Good | Not good |
| Example 8 | 7 | 3 | 0 | 0 | 0 |
| Example 2 | 3 | 6 | 1 | 0 | 0 |
| Example 3 | 1 | 5 | 4 | 0 | 0 |
| Comparative example 1 | 0 | 0 | 3 | 5 | 2 |
| Comparative example 2 | 0 | 1 | 7 | 2 | 0 |
| Comparative example 3 | 0 | 4 | 6 | 0 | 0 |

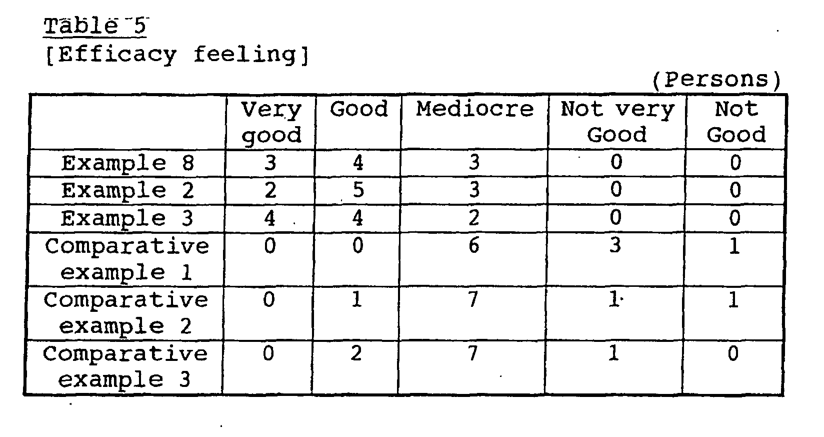

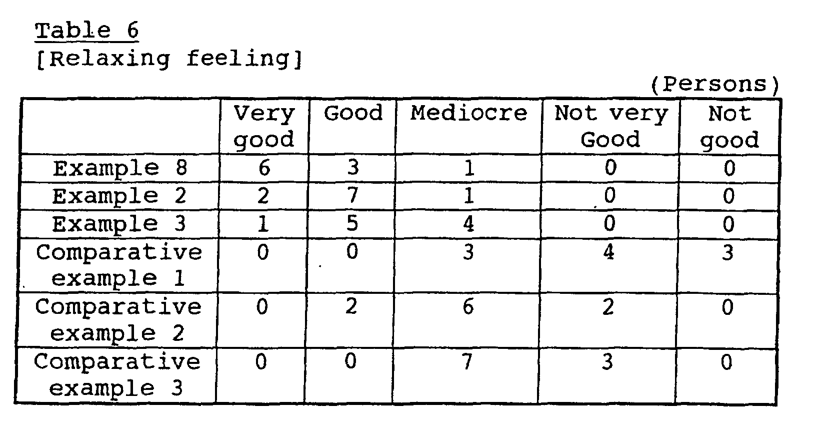

As shown in Tables 4-6 all examples 2,3 and 8 were excellent in the refreshing feeling, the efficacy feeling and the relaxing feeling when using.

### (Test example 4) Skin safety test

As for examples 1, 2 and 3 and comparative examples 1 and 3 the skin safety test was carried out. A 48-hr closed patch test was carried out for 30 healthy male and female persons. Changes of the skin were observed after 1 hr. and 24 hrs. from the removal, and the irritancy degree of the skin was evaluated according to the below standards. The results are shown in Table 7.
-: No change is observed in the skin
±: Faint flare in the skin
+: Clear flare in the skin
++: Severe feeling in the skin

**Table 7**

| Time After removal | Judgement Samples | + + | + | ± | - | Total (persons) | Positive ratio (%) |
|---|---|---|---|---|---|---|---|
| | | | | | | | Not less than ± |
| After 1 hr. | Example 1 | 0 | 0 | 0 | 30 | 30 | 0.0 |
| | Example 2 | 0 | 0 | 0 | 30 | 30 | 0.0 |
| | Example 3 | 0 | 0 | 1 | 29 | 30 | 3.3 |
| | Comparative example 1 | 0 | 0 | 3 | 27 | 30 | 10.0 |
| | Comparative example 3 | 0 | 0 | 6 | 24 | 30 | 20.0 |
| After 24 hrs | Example 1 | 0 | 0 | 0 | 30 | 30 | 0.0 |
| | Example 2 | 0 | 0 | 0 | 30 | 30 | 0.0 |
| | Example 3 | 0 | 0 | 0 | 30 | 30 | 0.0 |
| | Comparative example 1 | 0 | 0 | 1 | 29 | 30 | 3.3 |
| | Comparative example 3 | 0 | 0 | 3 | 27 | 30 | 10.0 |

As shown Table 7 examples 1-3 in the invention showed completely no or almost no irritancy.

### Industrial Applicability

The sheet-type pack of the invention has appropriate stickiness and good usability and is excellent in the skin safety. Because it is also excellent in a sebum-removal action and a sebum-suppressive effect to the skin and a relaxing effect by the refreshing agent and the perfume, it can satisfy relaxation needs of users. Therefore, the sheet-type pack of the invention can be applied in the fields of quasi-drug or cosmetics, which are used for skindressing and beautification, so that it is industrially very useful.

## Claims

1. A sheet-type pack prepared by adding one or more species selected from the group consisting of refreshing agents, perfumes and sebum-controlling agents to a base comprising a water-soluble polymer, a polyhydric alcohol, a crosslinking agent, water and a skin-care component.

2. The sheet-type pack according to claim 1, in which the water-soluble polymer comprises one or more species selected from the group consisting of gelatin, polyacrylic acid, partial neutralization products of polyacrylic acid and polyacrylic acid salts.

3. The sheet-type pack according to claims 1 or 2, in which the polyhydric alcohol comprises one or more species selected from the group consisting of polyethylene glycol and polypropylene glycol.

4. The sheet-type pack according to any one of claims 1 to 3, in which the cross-linking agent comprises one or more species selected from the group consisting of a slightly water-soluble aluminum compound and a polyfunctional epoxy compound.

5. The sheet-type pack according to any one of claims 1 to 4, comprising as the refreshing agent one or more species selected from the group consisting of peppermint oil, d-camphor, dl-camphor, l-menthol, isoplegol, 3-L-menthoxypropane-1,2-diol, menthyl pyrrolidonecarboxylate and L-menthyl-3-hydroxybutylate.

6. The sheet-type pack according to any one of claims 1 to 5, comprising as the perfume one or more species selected from a group consisting of eucalyptus oil, dl-menthol and clove oil.

7. The sheet-type pack according to any one of claims 1 to 6, comprising as the sebum-controlling agent clove oil.

8. The sheet-type pack according to any one of claims 1 to 7, wherein a mixing amount of the refreshing agent, the perfume and/or the sebum-controlling agent is 0.001-3 wt.% of the total amount of the base.

9. The sheet-type pack according to any one of claims 1 to 8, wherein an antiseptic is added.

10. The sheet-type pack according to any one of claims 1 to 9, which is applied to the face.

11. The sheet-type pack according to any one of claims 1 to 10, wherein the sebum component content after use is reduced not more than 40% of that before use.
